Europäisches Patentamt

**European Patent Office**

**Office européen des brevets**

(11) Numéro de publication: **0 092 076**

**B1**

## (12) FASCICULE DE BREVET EUROPÉEN

(45) Date de publication du fascicule du brevet:
17.12.86

(21) Numéro de dépôt: **83103149.7**

(22) Date de dépôt: **30.03.83**

(51) Int. Cl.⁴: **A 23 D  5/00,** A 23 L  1/36,
A 61 K  31/23, A 61 K  35/78 //
A23C11/04

(54) Composition lipidique destinée à l'alimentation orale, entérale ou parentérale.

(30) Priorité: **16.04.82  CH 2314/82**

(43) Date de publication de la demande:
**26.10.83 Bulletin 83/43**

(45) Mention de la délivrance du brevet:
**17.12.86 Bulletin 86/51**

(84) Etats contractants désignés:
**AT BE CH DE FR GB IT LI LU NL SE**

(56) Documents cité:
**FR-A-2 197 605**
**FR-A-2 255 055**
**GB-A-2 084 172**

(73) Titulaire: **SOCIETE DES PRODUITS NESTLE S.A.,
Case postale 353, CH- 1800 Vevey (CH)**

(72) Inventeur: **Ingenbleek, Yves, Chemin de la Paix 33,
CH- 1802 Corseaux (CH)**
Inventeur: **Traitler, Helmut, Route de St.- Légier
10a, CH- 1800 Vevey (CH)**
Inventeur: **Wessely, Jean- Yves, Avenue Bel- Air 7,
F-92270 Bois- Colombes (FR)**

EP 0 092 076 B1

**Description**

La présente invention a pour objet une composition lipidique destinée à l'alimentation orale, entérale ou parentérale.

De nombreuses situations pathologiques chez l'homme nécessitent un apport important de lipides pour remédier aux anomalies du métabolisme lipidique ou pour compenser un déficit spécifique (p.ex. troubles divers de la digestion et de la nutrition, besoins spécifiques en réanimation, dans les chocs et les soins intensifs).

On a mentionné, p.ex. dans la demande de brevet français No. 2.490.631, des compositions lipidiques destinées à être utilisées dans ce type de situation comprenant une huile contenant de l'acide gamma-linolénique ($C_{18}:3\omega6$) en mélange avec des esters de glycérol d'acides gras en $C_6$-$C_{12}$. L'huile contenant de l'acide gamma-linolénique proposée provient de graines d'<u>Oenothera biennis</u> et d'<u>Oenothera Lamarckiana</u> (onagre ou "evening primrose"), seule source disponible actuellement.

De plus, les esters de glycérol d'acide gras en $C_6$-$C_{12}$ constituent au moins 50% en poids de la composition lipidique. Une telle composition ne répond pas de manière optimale aux besoins mentionnés ci-dessus du fait qu'elle comporte un excès de triglycérides en $C_6$-$C_{12}$ qui sont connus pour être fortement cétogènes, et qu'elle ne contient pas de fractions lipidiques en $C_{12}$-$C_{18}$ qui sont cependant indispensables à l'équilibre nutritionnel au cours d'une alimentation prolongée des malades graves.

Dans la demande de brevet français No. 2.197.605, on a décrit entre autres compositions d'usage diététique ou cosmétique, un melange lipidique contenant des proportions mineures d'huile de noix de coco et d'huile d'onagre ou de bourrache ainsi qu'une proportion majeure d'huile de tournesol. Ici encore l'acide gamma-linolénique est présent sous forme de triglycérides dans l'huile d'onagre ou de bourrache extraites de graines de plantes dont la disponibilité est faible.

L'objet de l'invention est une composition lipidique ne présentant pas les inconvénients des compositions connues et qui permet l'apport journalier de lipides fournissant:

- des substrats énergétiques caloriques pour le fonctionnement équilibré du métabolisme interne,

- des acides gras essentiels dont le rôle est de participer à la régulation de nombreux métabolismes et structures cellulaires parmi lesquels la composition des membranes et la synthèse de nombreuses substances actives (prostaglandines, leucotriènes et thromboxanes).

La composition lipidique selon l'invention est caractérisée par le fait qu'elle est constituée par:

a) de 10 à 30 % en poids d'huile de pépins de fruits du genre Ribes contenant de l'acide gamma-linolénique sous forme de triglycérides,

b) de 20 à 40 % en poids de triglycérides d'acides gras en $C_6$-$C_{12}$ et

c) de 50 à 70 % en poids d'une huile contenant des triglycérides d'acides gras dont 80 % au moins sont des acides gras en $C_{12}$ à $C_{18}$, la somme des composants a) et b) ne dépassant pas 50 % en poids de la composition.

L'huile de pépins de fruits du genre <u>Ribes</u> contient au moins 4 % en poids d'acide gamma-linolénique sous forme de triglycérides. Celle-ci peut être obtenue par extraction par solvant à partir des pépins de fruits du genre <u>Ribes</u>, c'est-à-dire en pratique de cassis (<u>Ribes nigrum</u>), de groseilles (<u>Ribes rubrum</u>) et de groseilles à maquereau (<u>Ribes ova-crispa</u> ou <u>grossularia</u>) ou d'hybrides de ces fruits, p. ex. selon le procédé décrit dans la demande de brevet européen no. 92 085. Ces pépins se trouvent dans les résidus de la fabrication des jus, des confitures et gelées ou des eaux-de-vie, liqueurs et schnaps.

Leur contenu en lipides est de 12 à 30 % en poids suivant la matière première. La phase lipidique contient à son tour de 4 à 19 % en poids d'acide gamma-linolénique.

A titre indicatif, l'huile des pépins de ces fruits est constituée des acides gras suivants, en poids:

| Acides gras | Cassis | Groseille | Groseille à maquereau |
|---|---|---|---|
| C 16 : 0 | 6 – 7 % | 4 – 5 % | 7 – 8 % |
| C 18 : 0 | 1 – 2 % | 1 – 2 % | 1 – 2 % |
| C 18 : 1 cis | 9 – 10 % | 14 – 15 % | 15 – 16 % |
| C 18 : 1 trans | 0,5 % | 0,5 – 1 % | 1 – 2 % |
| C 18 : 2 ω 6 | 47 – 49 % | 41 – 42 % | 39 – 41 % |
| C 18 : 3 ω 6 | 15 – 19 % | 4 – 5 % | 10 – 12 % |
| C 18 : 3 ω 3 | 12 – 14 % | 29 – 31 % | 19 – 20 % |
| C 18 : 4 ω 3 | 3 – 4 % | 2,5 – 3,5 % | 4 – 5 % |

L'huile de cassis qui est préférée à cause de sa teneur élevée en acide gamma-linolénique, contient en outre de 1 à 2 % en poids de corps insaponifiables, tels que alcools aliphatiques, hydrocarbures, tocophérols, squalène, βsitostérol, campestérol et Δ-7 stigmastérol.

L'acide gamma-linolénique est un acide gras polyinsaturé qui est métabolisé par l'organisme en prostaglandines via l'acide dihomo-gamma-linolénique et l'acide arachidonique (5, 8, 11, 14 - éicosatétraénoique), qui est lui -même un constituant des membranes cellulaires tandis que l'acide alpha-linolénique ne contribue pas de la même manière à ce processus métabolique. La conversion acide linoléique (C 18: 2 ω 6) → acide gamma-linolénique dans les tissus est incomplète (4 - 20 % comparé à 90 - 98 % pour la transformation acide gamma-linolénique → acide arachidonique) et peut même être inexistante en cas d'absence ou d'inactivation de l'enzyme Δ-6-désaturase. L'acide gamma-linolénique devient donc essentiel dans les situations pathologiques (p. ex. stress, affections médicales et chirurgicales graves, cancer, prématurité, senescence, etc...) où il est démontré que cet enzyme est déficient. Un manque d'acides gras essentiels conduit en effet à une carence nutritionnelle affectant tous les processus métaboliques mentionnés plus haut et pouvant conduire à des désordres biochimiques ou à des lésions organiques (p. ex. troubles de la coagulation, lésions dermatologiques, troubles endocriniens, lésions myocardiques, troubles hépatiques, articulaires, neurologiques et mentaux). On voit donc l'intérêt que présente un apport d'acide gamma-linolénique pour la prévention ou pour le traitement de ces anomalies.

Les triglycérides d'acides gras en $C_6$-$C_{12}$ et en particulier en $C_8$-$C_{10}$, appelés communément "triglycérides à chaîne moyenne", qu'ils soient préparés industriellement ou extraits à partir de produits naturels, d'une espèce de Cuphea contenant 80 à 90 % d'acide gras en $C_{10}$, constituent un apport énergétique immédiat. Industriellement, ils sont obtenus à partir des graisses de coco et de palmiste par hydrolyse, fractionnement des acides gras, essentiellement en $C_8$-$C_{10}$, par distillation et reestérification de ceuxci avec le glycérol.

Les acides gras apportés par l'alimentation, une fois traversé le tube digestif, ou directement lorsqu'ils sont injectés par voie intraveineuse, sont utilisés au niveau des sites cataboliques (ou éventuellement stockés). Ils subissent une oxydation au niveau de la mitochondrie (β-oxydation) aboutissant à la formation de radicaux acétyles qui entrent dans le cycle de Krebs ou, en cas de manque en acide oxaloacétique, sont transformés en corps cétoniques (acide hydroxybutyrique et acide acétoacétique) au niveau du foie. Ainsi, ils fourniront une importante quantité d'énergie maintenant les grandes fonctions de synthèse de l'organisme.

Dans certaines situations pathologiques, les lipides ingérés ne sont pas ou insuffisamment absorbés par le tube digestif (insuffisance hépatique, insuffisance de sécrétion biliaire, insuffisance pancréatique, onsuffisance fonctionnelle ou organique de l'intestion grêle).

Dans ces situations, il est indiqué de fournir des triglycérides à chaîne moyenne qui sont absorbés par l'intestin de manière beaucoup plus aisée que les acides gras longs.

Par ailleurs, les triglycérides à chaîne moyenne pénètrent directement dans la mitochondrie (site d'oxydation). Ils n'ont pas besoin d'un système vecteur spécifique qui permet le transfert des acides gras à longue chaîne. Enfin, des travaux récents ont mis en évidence un effet d'épargne des triglycérides à chaîne moyenne sur les acides gras polyinsaturés. (S.C. Frost and M.A. Wells, Archives of Diochemistry and Biophysics, 1981, 211, 2, 537-546; D. Sailer and M. Muller, Medium chain triglycerides in parenteral nutrition, J.P.E.N., 1981, 5, 2, 115-119). Ainsi, les triglycérides à chaîne moyenne sont oxydés préférentiellement, préservant les acides gras polyinsaturés de l'oxydation, pour leurs fonctions de synthèse et de structure.

La composition lipidique selon l'invention contient également une huile participant à l'apport énergétique et fournissant au moins 80 % d'acides gras en $C_{12}$-$C_{18}$, calculé sur le nombre total d'acides gras, sous forme de triglycérides.

Ce peut être une huile d'origine animale ou végétale fournissant notamment les acides palmitique, stéarique, oléique, linoléique et α-linolénique.

Pour l'utilisation orale ou entérale, l'huile sera une huile végétale couramment utilisée en nutrition comme p. ex. l'huile de carthame, de blé, de tournesol, d'arachide, de coton et plus particulièrement l'huile de mais, de soja et l'huile de pépins de raisins. On peut également utiliser une huile d'origine animale comme p. ex. la matière grasse lactique anhydre, l'huile de beurre ou des fractions de celle-ci fournissant au moins 80 % d'acides gras en $C_{12}$-$C_{18}$, calculé sur le nombre total d'acides gras. On peut naturellement utiliser un mélange des huiles précédentes.

Pour l'utilisation parentérale, on préférera une huile végétale particulièrement riche en fractions insaturées fournissant notamment les acides oléique, linoléique et αlinolénique comme p. ex. l'huilé de soja ou l'huile de pépins de raisin.

L'huile de soja contient les acides gras suivants en poids:

| Acides gras | % |
|---|---|
| C 16 : 0 | 9,9 – 12,1 |
| C 16 : 1 ω 7 | 0,2 – 0,4 |
| C 18 : 0 | 1,3 – 4,8 |
| C 18 : 1 ω 9 | 22,5 – 31,2 |
| C 18 : 2 ω 6 | 46,9 – 54,7 |
| C 18 : 3 ω 3 | 5,2 – 8,5 |

L'huile de pépins de raisins contient les acides gras suivants en poids:

| Acides gras | % |
|---|---|
| C 16 : 0 | 6,7 |
| C 18 : 0 | 3,9 |
| C 18 : 1 cis | 14,2 |
| C 18 : 1 trans | 0,7 |
| C 18 : 2 ω 6 | 74,2 |
| C 18 : 3 ω 3 | 0,3 |

L'acide $\alpha$-linolénique (C 18: 3 ω 3) ne peut être synthétisé par les mammifères, mais il ne semble pas réduire les lésions induites chez l'animal par la carence en acides gras essentiels. En fait, une publication récente (R.T. Holman, S.B. Johnson, T.F. Hatch, A case of human linolenic acid deficiency involving neurological abnormalities, The Amer. Journal of Clinical Nutrition, 1982, 35, 617-623), décrit le cas d'un jeune enfant recevant une perfusion lipidique sans acide $\alpha$-linolénique qui développa des signes de carence en cet acide gras (torpeur, paresthésies, retard à l'apprentissage de la marche, douleurs des membres inférieurs) qui furent totalement dégressifs lorsque l'on ajouta l'acide $\alpha$-linolénique au régime alimentaire parentéral).

Il faut noter que l'acide $\alpha$-linolique est précurseur des prostaglandines de la série 3 par l'acide eicosapentaénoïque ($C_{20}$: 5 ω 3) dont il permet la synthèse.

Il est généralement admis que le transport intracellulaire de ces fractions en $C_{12}$-$C_{18}$ est assuré par un vecteur spécifique appelé carnitine dont la synthèse endogène est assurée à partir de la lysine et de la méthionine apportées par l'alimentation. Dans les états phatologiques susmentionnés il est possible que la synthèse de la carnitine soit déprimée. Dans ces cas un apport exogène de carnitine par voie entérale ou parentérale pourrait être d'un grand bénéfice pour le malade. On peut donc ajouter celle-ci à la composition lipidique si l'état du malade l'exige.

Les besoins minima d'un adulte en bonne santé
- en acide linoléique sont de l'ordre de 3 % de la ration calorique journalière
- en acide $\alpha$-linolénique de l'ordre de 0,5 % de la ration calorique journalière
- en acide gamma-linolénique d'environ 2 g/24 heures.

Lorsqu'elle est destinée à l'alimentation parentérale et compte tenu de ces différentes considérations relatives à l'absorption des acides gras essentiels et des triglycérides à chaîne moyenne, la composition lipidique de l'invention a de préférence la constitution suivante:

a) de 10 à 20 % en poids de l'huile contenant de l'acide gamma-linolénique sous forme de triglycérides,

b) de 20 à 30 % en poids de triglycérides à chaîne moyenne et

c) de 60 à 70 % en poids d'une huile végétale contenant les fractions lipidiques en $C_{12}$ à $C_{18}$ et notamment les acides oléique, linoléique et $\alpha$-linolénique sous forme de triglycérides, la somme des composants a) et b) ne dépassant pas 40 % en poids de la composition.

Les compositions lipidiques selon l'invention seront présentées sous une forme adaptée au mode d'administration.

4

Lorsqu'elles sont destinées à l'alimentation orale ou entérale, elles peuvent être p. ex. sous forme de capsules ou gélules contenant le mélange lipidique ou se présenter sous forme d'émulsions dans lesquelles les lipides sont émulsifiés en présence d'une phase aqueuse contenant p. ex. des protéines, des acides aminés, des hydrates de carbone, des sels minéraux et des vitamines et si nécessaire des antioxydants et des émulsifiants. De telles émulsions seront avantageusement séchées sous forme de poudre qu'on peut reconstituer sous forme liquide par dispersion dans l'eau au moment de l'utilisation.

Pour l'administration parentérale, les compositions lipidiques se présenteront sous forme d'émulsions stabilisées physiquement (finesse des particules lipidiques) et chimiquement (protection contre l'oxydation p. ex. avec les tocophérols). Sous cette forme, les lipides seront émulsifiés avec des phosphatides de soja, d'oeuf, des fractions de ceux-ci ou tout autre émulsifiant naturel. Les émulsions pourront contenir des hydrates de carbone tels que p. ex. le glucose, polyols - p. ex. le xylitol, le sorbitol ou le glycérol et/ou des acides aminés ou des protéines (p. ex. la carnitine) dans leur phase aqueuse. Les émulsions obtenues seront isotoniques ou hypertoniques, apyrogènes et se présenteront sous forme stérile.

Les exemples suivants dans lesquels les pourcentages sont pondéraux sauf indication contraire, illustrent l'invention:

**Exemple 1**

On prépare une émulsion aqueuse isotonique pour l'alimentation parentérale à partir des lipides suivants:

|  | % en poids |
|---|---|
| – huile de cassis fournissant 2 % en poids d'acide γ-linolénique | 12 |
| – triglycérides à chaîne moyenne | 20 |
| – huile de soja | 68 |

On mélange les lipides et on y ajoute 6 % de phosphatides de soja comme émulsifiant, calculé sur le poids de lipides et on stérilise le mélange.

On y ajoute le complément d'eau stérile en quantité suffisante pour obtenir 100 g de lipides par litre d'eau après émulsification.

On émulsifie, de préférence en circuit fermé dans des conditions aussi proches que possible de la stérilité, dans un homogénéisateur à une température inférieure à la température d'inversion de phase, p. ex. de 20 à 60°C et de préférence à 55°C, à 140-180 bars jusqu'à ce que les particules lipidiques soient de dimension inférieure à 0,5 µm.

On remplit des flacons de 1 litre dans des conditions aseptiques et on les stérilise pendant 20 à 30 min à 110-130°C.

On peut en variante réaliser l'émulsification dans un moulin colloïdal.

**Exemple 2**

On procède comme à l'exemple 1 pour préparer une émulsion hypertonique pour l'alimentation parentérale contenant du xylitol, du sorbitol et du glycérol à la place du glucose dans des quantités résultant en une concentration finale de 25, 50 et 100 g/l respectivement, en ajoutant l'un de ces composés à l'eau avant émulsification.

**Exemple 3**

On prépare une émulsion hypertonique pour l'alimentation parentérale à partir des ingrédients suivants en utilisant le mode opératoire indiqué dans l'exemple 1:
triglycérides à chaîne moyenne 20 g
Lipides huile de pépins de raisins 60 g
huile de pépins de cassis (apportant 2 g d'acide gamma-linolénique) 12 g
Emulsifiant: phosphatides d'oeuf 8 g
Carnitine (dans la phase aqueuse) 2 g
Glucose (dans la phase aqueuse) 50 g
Eau stérile, complément à 1 litre

**Exemple 4**

On prépare une émulsion hypertonique pour l'alimentation parentérale à partir des ingrédients suivants en utilisant le mode opératoire indiqué dans l'exemple 1:

triglycérides à chaîne moyenne 40 g
Lipides huile de pépins de raisins 120 g
huile de pépins de cassis (apportant 4 g d'acide gamma-linolénique) 24 g
Emulsifiant: phosphatides d'oeuf 16 g
Glucose (dans la phase aqueuse) 50 g
Eau stérile, complément à 1 litre

**Exemple 5**

On prépare une poudre de lait pour prématurés comme indiqué p. ex. dans le brevet français No. 2.388.503, à partir des ingrédients suivants:

huile de pépins de cassis (fournissant Lipides 0,4 g d'acide gamma-linolénique) 2,4 g
triglycérides à chaîne moyenne 9,6 g
graisse lactique anhydre 12,0 g
Hydrates de carbone dont 41,6 g de lactose et 15,3 g de glucose 56,9 g
Protéines dont 4,4 g de caséine et 10 g de protéines de lactosérum 14,4 g
Cendres 1,7 g
dont
Calcium 350 mg
Phosphore, 200 mg
Potassium 370 mg
Sodium 103 mg
Eau 3,0 g
ainsi que les quantités minima des vitamines et oligoéléments ci-après:

| | |
|---|---|
| Fer | 6,0 mg |
| Cuivre | 0,3 mg |
| Zinc | 1,8 mg |
| Iode | 25 µg |
| Acide folique | 80 µg |
| Vitamine C | 200 mg |
| Vitamine E | 10 mg |
| Vitamine $B_1$ | 0,35 mg |

Pour reconstituer le produit, on disperse 15 g de poudre dans 90 ml d'eau.

**Exemple 6**

On prépare une composition en poudre pour l'alimentation entérale des vieillards à partir des ingrédients suivants:

**Lipides**

Huile de cassis fournissant 2,4 kg d'acide 36 kg γ-linolénique
Triglycérides à chaîne moyenne 24 kg
Huile de soja 60 kg

6

### Protéines

Lactalbumine de lactosérum dégradée par la pancréatine contenant 20 % d'amino-acides libres et 18 % de peptides de poids moléculaire inférieur à 1000 117,5 kg

### Hydrates de carbone

Dextrine-maltose 270 kg
Saccharose 94,5 kg
Amidon prégélifié 10 kg
Sels minéraux et vitamines selon besoin
On dissous 10 kg d'amidon prégélatinisé dans 333 kg d'eau froide. On dissout séparément 117,5 kg de lactalbumine dégradée en poudre dans 500 kg d'eau à 65°C et on y ajoute les sels minéraux puis la solution d'amidon, 270 kg de dextrine-maltose, 94,5 kg de saccharose et les vitamines, celles-ci sous forme d'une solution aqueuse à 10 %. A cette phase aqueuse on ajoute 120 kg du mélange lipidique préalablement chauffé à 65°C, On brasse les deux phases, On les fait passer dans un moulin colloïdal et ensuite dans un homogénéisateur à 65°C et sous une pression de 300 bars. On pasteurise le mélange homogénéisé à 105°C pendant 1 min, on le refroidit à 65°C et on l'homogénéise à nouveau sous une pression de 400 bars. On sèche ensuite le mélange par atomisation et on conditionne la poudre dans des récipicnts étanches à l'air gazés avec de l'azote. On reconstitue le produit en dissolvant 15 g de celui-ci dans 90 ml d'eau.

### Revendications

1. Composition lipidique caractérisée par le fait qu'elle est constituée par
a) de 10 à 30 % en poids d'huile de pépins de fruits du genre <u>Ribes</u> contenant de l'acide gamma-linolénique sous forme de triglycérides,
b) de 20 à 40 % en poids de triglycérides d'acides gras en $C_6$-$C_{12}$ et
c) de 50 à 70 % en poids d'une huile contenant des triglycérides d'acides gras dont 80 % au moins sont des acides gras en $C_{12}$ à $C_{18}$, la somme des composants a) et b) ne dépassant pas 50 % en poids de la composition.
2. Composition lipidique selon la revendication 1, caractérisée par le fait qu'elle est constituée par
a) de 10 à 20 % en poids d'huile de pépins de fruits du genre <u>Ribes</u>,
b) de 20 à 30 % en poids de triglycérides à chaîne moyenne et
c) de 60 à 70 % en poids d'une huile végétale contenant les fractions lipidiques en $C_{12}$ à $C_{18}$ et notamment les acides oléique, linoléique et α-linolénique sous forme de triglycérides, la somme des composants a) et b) ne dépassant pas 40 % en poids de la composition.
3. Composition lipidique selon la revendication 3, caractérisé par le fait que l'huile contenant l'acide γ-linolénique est l'huile de pépins de cassis.
4. Composition lipidique selon l'une quelconque des revendications 1 à 3, caractérisée par le fait que l'huile contenant les fractions lipidiques en $C_{12}$ à $C_{18}$ est l'huile de soja ou l'huile de pépins de raisin.
5. Composition lipidique selon la revendication 3, caractérisée par le fait qu'elle contient 12 % d'huile de cassis, 20 % de triglycérides à chaîne moyenne et 68 % d'huile de soja calculé sur le poids de lipides.
6. Composition lipidique selon l'une quelconque des revendications 1 à 5 sous une forme adaptée à l'alimentation orale, entérale ou parentérale.
7. Médicament contenant la composition lipidique selon l'une quelconque des revendications 1 à 5.

### Patentansprüche

1. Lipidzusammensetzung, dadurch gekennzeichnet, daß sie gebildet ist aus
a) 10 bis 30 Gew.-% Öl von gernen der Früchte der Gattung Ribes mit einem Gehalt an Gamma-Linolensäure in Form von Triglyceriden,
b) 20 bis 40 Gew.-% Triglyceriden von $C_6$-$C_{12}$-Fettsäuren und
c) 50 bis 70 Gew.-% eines Öles mit einem Gehalt an Triglyceriden von Fettsäuren, von denen wenigstens 80 % $C_{12}$ - $C_{18}$-Fettsäuren sind, wobei die Summe der kompopenten a) und b) 50 Gew.-% der Zusammensetzung nicht übersteigt.
2. Lipidzusammensetzung nach Anspruch 1, dadurch gekennzeichnet, daß sie gebildet ist aus
a) 10 bis 20 Gew.-% Öl von Bernen der Früchte der Gattung Ribes,
b) 20 bis 30 Gew.-% Triglyceriden mit mittlerer Fettenlänge und
c) 60 bis 70 Gew.-% eines Pflanzenöles, das die $C_{12}$-$C_{18}$-Lipidfraktionen und insbesondere Ölsäure, Linolsäure und α-Linolensäure in Form von Triglyceriden enthält, wobei die Summe der Komponenten a) und b) 40 Gew.-

% der Zusammensetzung nicht übersteigt.

3. Lipidzusammensetzung nach Anspruch 1, dadurch gekennzeichnet, daß das die Gamma-Linolensäure enthaltende Öl das Öl von gernen von Cassis (schwarze Johannisbeere) ist.

4. Lipidzusammensetzung nach einem der Ansprüche 1 bis 3, dadurch gekennzeichnet, daß das die $C_{12}$ - $C_{18}$-Lipidfraktionen enthaltende Öl Sojaöl oder Traubenkernöl ist.

5. Lipidzusammensetzung nach Anspruch 3, dadurch gekennzeichnet, daß sie 12 % Cassisöl, 20 % Triglyceride mit mittlerer gettenlänge und 68 % Sojaöl, bezogen auf das Gewicht der Lipide, enthält.

6. Lipidzusammensetzung nach einem der Ansprüche 1 bis 5 in einer zur oralen, enteralen oder parenteralen Ernährung angepaßten Form.

7. Medikament mit einem Gehalt an der Lipidzusammensetzung nach einem der Ansprüche 1 bis 5.

## Claims

1. A lipid composition, characterized in that it comprises (a) from 10 to 30% by weight of an oil containing $\gamma$-linolenic acid in the form of triglycerides, said oil being obtained from the pips or seeds of fruits of the genus <u>Ribes</u>, (b) from 20 to 40% by weight of triglycerides of $C_6$-$C_{12}$ fatty acids, and (c) from 50 to 70% by weight of an edible oil containing fatty acids in the form of triglycerides, at least 80% of the number of the fatty acids present being $C_{12}$ to $C_{18}$ fatty acids, the total of components (a) and (b) not exceeding 50% by weight of the composition.

2. A lipid composition according to claim 1, characterized in that it comprises (a) from 10 to 20% by weight of oil obtained from the pips of fruits of the genus <u>Ribes</u>, (b) from 20 to 30% by weight of medium chain triglycerides, and (c) from 60 to 70% by weight of a vegetable oil containing $C_{12}$-$C_{18}$ fatty acids and especially the oleic, linoleic and $\alpha$-linolenic acids in the form of triglycerides, the total of components (a) and (b) not exceeding 40% by weight of the composition.

3. A lipid composition according to claim 1, characterized in that oil containing $\gamma$-linolenic acid is black currant oil.

4. A lipid composition according to any of claims 1 to 3, characterized in that the oil containing $C_{12}$ to $C_{18}$ fatty acid triglycerides is grape seed oil or soya oil.

5. A lipid composition according to claim 3, characterized in that it comprises 12% of black currant oil, 20% medium chain triglycerides and 68% soya oil, calculated on the weight of lipids.

6. A lipid composition according to any of claims 1 to 5, adapted for oral, enteral or parenteral administration.

7. A drug comprising a lipid composition according to any of claims 1 to 5.